# EUROPEAN PATENT SPECIFICATION

(11) **EP 2 461 856 B1**
(45) Date of publication and mention of the grant of the patent: **18.05.2016**
(21) Application number: 10748138.4
(22) Date of filing: 29.07.2010
(51) Int. Cl.: A61M 5/44, A61M 5/148

(54) **THERMOSTATTING DEVICE FOR CONTAINERS OF BIO-MEDICAL FLUIDS FOR PARENTERAL ADMINISTRATION, PARTICULARLY BAGS FOR MEDICAL FLUIDS, BLOOD OR THE LIKE**
THERMOSTATISIERUNGSVORRICHTUNG FÜR BEHÄLTER FÜR BIOMEDIZINISCHE FLÜSSIGKEITEN FÜR PARENTERALE VERABREICHUNG, IM BESONDEREN BEUTEL FÜR MEDIZINISCHE FLÜSSIGKEITEN, BLUT ODER DERGLEICHEN
DISPOSITIF DE THERMOSTATISATION POUR RÉCIPIENTS DE FLUIDES BIOMÉDICAUX PAR VOIE PARENTÉRALE, EN PARTICULIER POCHES POUR FLUIDES MÉDICAUX, SANG, ETC.

(30) Priority: 06.08.2009 IT MO20090207
(43) Date of publication of application: 13.06.2012
(73) Proprietor: Sidam S.r.l., 41037 S. Giacomo Roncole, Mirandola (MO) (IT)
(72) Inventor: AZZOLINI, Graziano, 41032 Cavezzo (IT)
(74) Representative: Brunacci, Marco
(86) International application number: PCT/IB2010/001875
(87) International publication number: WO 2011/015912

(56) References cited:
- EP-A1- 1 747 788
- WO-A1-88/07384
- WO-A1-98/31312
- WO-A1-2005/072666
- WO-A1-2005/115503
- WO-A1-2009/086684
- FR-A- 1 281 309
- US-A- 3 902 635
- US-A- 5 328 477
- US-A1- 2008 147 016

## Description

### Technical Field

The present invention relates to a thermostatting device for containers of bio-medical fluids for parenteral administration, particularly bags for medical fluids, blood or the like.

### Background Art

It is known that people with health problems, affected by attacks, victims of accidents must undergo medical treatment which, sometimes, requires the parental administration of bio-medical fluids and/or liquids (medicinal, saline solutions, blood, etc.) at a preset temperature.

In some cases, e.g., the patients are in a state of shock characterised by hypovolaemia (volumetric reduction of blood) and hypothermia (lowering of body temperature), and have to be transfused fluids at a temperature generally between 33°C and 40°C.

To treat tumoural illnesses on the other hand, the need often arises to administer chemotherapy products, the effectiveness of which varies according to temperature and which, therefore, have to be infused at a temperature generally above room temperature.

Similar considerations can be made regarding the means of contrast used in diagnostics, these too administered at the patient's body temperature.

The fluids to be injected are currently packaged in containers of the flexible bag type, plastic or glass bottles or vials, which have a dispensing mouth from which the fluid can be taken by means of a standard syringe or by means of an extraction and conveying duct, commonly called "infusate source", the outlet extremity of which is associated with an element of injection (catheter or needle) to the patient.

To condition the temperature of fluids, particular systems are usually used for heating the fluid containers and/or the fluid extraction and conveying duct.

The use is known, e.g., of devices essentially composed of a box inside which the containers are placed connected to the patient by means of the infusate sources and which, during the dispensing of the fluid, is heated by means of heat production means, e.g., of the type of heating elements; such box also has an electronic temperature control and adjustment circuit.

Other devices of known type heat the extraction and conveying duct during the administration and essentially consist in a metal element which is coupled to heating elements, supplied with current, and which is placed close to the section of the infusate source to be conditioned; in this case as well, an electronic circuit controls and regulates the reached temperature.

These heating devices for heating the container and/or the extraction and conveying duct do have a number of drawbacks among which should be recalled the fact that they have far from negligible weight and overall dimensions that make their transport and use particularly difficult and hard, they are structurally and constructively complex, they require the intervention of skilled operators and they have far from negligible costs.

Fluid container heating or cooling devices are also known which, in slang, are called of the so-called "chemical" type inasmuch as they exploit the heat produced by an exothermic or endothermic chemical reaction between two or more reagent substances.

These "chemical" type devices are essentially made up of a wrapping, of the type of a bag, split into two or more compartments which are separated the one from the other by a tearable membrane and of which one contains a first component and the other a second component suitable for reacting together with an exothermic or endothermic reaction depending on the type of use to which such devices are to be put.

At the time of use, an operator acts manually on the wrapping, applying on this such a pressure as to break the tearable membrane and allow the chemical mixing of the components, and then manually places the device in contact with the container of the fluid to be administered to thermally condition it.

These "chemical" type devices also have a number of drawbacks, such as, e.g., the fact that they have to be stored, transported and handled with care to prevent them accidentally suffering knocks such as to accidentally activate at inopportune times the reaction between the chemical components which they contain, with the risk of prematurely exhausting their capacity to develop calories/frigories and thus become unusable when they are actually needed. Another drawback of the "chemical" type devices consists in the fact that they contain air which, having low heat conductivity, acts as insulation and reduces the exchange of heat with the fluid containers to be conditioned.

As an alternative to the systems and the devices described above, the use is known of particular heating appliances composed of an electric oven inside which the containers are placed to keep the fluid at the required temperature and from which they are removed only an instant before being used.

The use of such appliances, though very simple and functional, nonetheless allows administering the fluid at the required temperature only for a limited period of time inasmuch as, once taken out of the ovens, the containers quickly start to exchange heat with the outside and quickly tend to reach room temperature.

To overcome the above drawbacks of the state of the art, devices are known usable to support and thermally condition bio-medical fluid containers such as bottles or vials.

Such devices comprise, in particular, a cup-shaped support, having a top opening and a bottom opening, inside which can be placed, upside down, a container of the type of a bottle or a vial.

The cup-shaped support has an outer surface in heat insulating material and an inner surface in contact with the container, which is made of heat conductive material.

Such devices further comprise heating means of the inner surface, composed, e.g., of one or more heating elements.

Such devices, nevertheless, have a limited range of uses inasmuch as they can only be used to maintain the temperature and heat bio-medical fluids contained in bottles or other similar rigid containers and of substantially cylindrical shape. WO2009086684 discloses an extrusion apparatus for automatic-heating transfusion includes an openable box body, which includes an upper box body and a lower box body.

An upper pressure plate and a lower pressure plate are respectively provided in the upper box body and the lower box body, so as to accommodate and press a transfusion bag.

A heating and conducing unit is provided under the lower pressure plate, and a buzzer is provided between the upper pressure plate and the lower pressure plate.

WO2005072666 discloses a heating cabinet, which can be put vertical, including a housing for housing a bag, a flexible cover and including a beating plate inside the cabinet. It also includes an inflatable bellows or bladder disposed on the cover interior surface to apply pressure to the solution bag to achieve a desired solution flow or infusion rate.

FR1281309 describes a hangable box for containing a bag drainable by means of a tube, the bag resting at a vertical wall and being pressed by a mechanism for controlling the flow-rate comprising a spring acting on a blade hinged to a lever at an axis, the lever in turn being hinged at a suspension means.

EP1747788 discloses a device which comprises a hollow shell and is provided internally with a receptacle for at least one bag for containing a liquid which is provided with an opening for the outflow of the liquid. The device further includes presser means associated with the shell and adapted to compress the bag, and means for maintaining the temperature of the liquid, which are associated with the shell and are suitable alternatively to heat and cool the bag.

US5328477 discloses a liquid infusion system, placeable horizontal on a table top, including a flexible, flat, collapsible bag for storms liquid. A rigid housing encloses the bag with the outlet tube projecting therefrom. A spring biased rigid pivot plate is positioned within the housing. The plate exerts pressure on the bag, tending to urge the bag toward a flat collapsed position and to expel liquids through the outlet when any liquid is present in the bag.

WO8807384 describes an apparatus for heating and mixing transfusion or infusion liquid in a flexible liquid bag and discharging the liquid therefrom.

The apparatus has movable heater housings in which the liquid bag is kept squeezed between at least two support plates of which one constitutes a heating plate. Each heater housing is adapted to perform a reciprocatory rotary motion during heating the liquid, this motion having a large amplitude between two end positions. Furthermore, a flexible, expandable pressure bag is arranged for achieving a squeezing action against one side of the liquid brag. The pressure bag is connected to a pressurized medium source such that by a squeezing action it achieves liquid discharge directly from the liquidbagin the heater housing to an infusion apparatus or the like.

WO2005115503 discloses an automatic ressurized box for infusin liquid or food which can be used in disaster area salvage. It comprises an upper cover and a lower housing connected to the upper cover to form an enclosed box; also a pressure plate is provided for pressing a medicine bag. US2008147016 describes an apparatus for pressure infusion and temperature control of infused liquids includes a receptacle for receiving a liquid-filled bag containing intravenous solution and an inflatable pressure device.

The inflatable pressure device expands within the pressure device bag upon inflation by means of a bulb and exerts pressure on the liquid-filled bag. A heating element may be disposed on the inflatable pressure device bag to heat the liquid-filled bag to a desired temperature.

US3902635 discloses a fluid-dispensing apparatus comprising a housing including a movable plate-like piston and a unitary operating mechanism therefor including a shaft disposed within the housing and secured to the piston for retracting the piston to permit a fluid-filled bag to be inserted in a chamber thus formed in the housing and releasing the piston to apply pressure to the bag and to feed fluid therefrom.

A handle for operating the apparatus is disposed outside of the housing and that is recessed to minimize the size of the apparatus. A plate, of selected thickness, is removably inserted in the chamber in the housing in contact with the fluid-flled bag to permit the pressure of the piston to be adjusted to the volume of the bag. This plate is also usable as a heater for heating the fluid in the bag.

WO9831312 discloses a system for infusing a fluid into a patient. In one exemplary embodiment, the systems comprises a volume of fluid and a temperature altering device in close proximity to the volume of fluid. The temperature altering device is employed to heat or cool the volume of fluid to a desired temperature. A positive pressure device is provided to place the volume of fluid under positive pressure while at the desired temperature. A transfer member is further provided to transfer at least some of the fluid into the patient while at the desired temperature.

### Description of the Invention

The main object of the present invention, which is defined by claim 1, is to provide a support device for containers of bio-medical fluids for parental administration, particularly bags for medical fluids, blood or the like, that favours the infusion to patients of fluids brought to a preset temperature without requiring, during administration, complicated and cumbersome heating systems or devices.

A further object of the present invention is to provide a device that can be used in conditions of utmost safety for the patient and with utmost efficiency of use. Another object of the present invention is to be structurally and constructively simple, to be of reduced weight and overall dimensions and to also be easy to transport and handle by fairly inexpert health workers.

The above objects are all achieved by the present thermostatting device for containers of bio-medical fluids for parenteral administration, particularly bags for medical fluids, blood or the like, characterized by the fact that it comprises at least a container having at least one compartment for housing at least one flexible bag containing a bio-medical fluid, at least one portion of heat conductive surface suitable for being placed in contact with said bag when this is arranged inside said compartment, heating means for heating said heat conductive surface to bring and/or keep to a preset temperature the fluid inside said bag and retention means for retaining said bag inside said compartment which are suitable for keeping said bag in contact with said heat conductive surface during the emptying of the bag itself.

### Brief Description of the Drawings

Other characteristics and advantages of the present invention will become more evident from the description of two preferred, but not sole, embodiments of a thermostatting device for containers of bio-medical fluids for parenteral administration, particularly bags for medical fluids, blood or the like, illustrated purely as an example but not limited to the annexed drawings in which:
figure 1 is an axonometric view of a first embodiment of the device according to the invention;
figure 2 is a side and section view of the device according to the invention of figure 1;
figure 3 is a side and section view of a second embodiment of the device according to the invention;
figure 4 is an axonometric and partially exploded view of the first embodiment of the device according to the invention.

### Embodiments of the Invention

With reference to such figures, globally indicated by 1 is a thermostatting device for containers of bio-medical fluids for parenteral administration, which can be particularly used to support and heat flexible bags of the traditional type used for containing medical fluids, blood or similar substances.

The device 1 comprises:
- a container 2 having a housing compartment 3 of a flexible bag A containing a bio-medical fluid;
- a heat conductive surface 4 suitable for being placed in contact with the bag A when this is arranged inside the compartment 3;
- heating means 5 for heating the heat conductive surface 4 to bring and/or keep at a preset temperature the bio-medical fluid inside the bag, before or during administration to a patient;
- retention means for retaining the bag A inside the compartment 3 suitable for maintaining the bag in contact with the heat conductive surface 4 heated by means of the heating means 5, during the emptying of the bag itself.

More specifically, the retention means allow keeping a face of the bag A in direct contact with the heat conductive surface 4 heated during the administration of the bio-medical fluid to a patient. The outflow of the bio-medical fluid in fact leads to a reduction in the overall dimensions of the bag A and, consequently, the presence of such retention means allows avoiding the detachment of the bag A from the heat conductive surface 4.

In particular, with reference to the embodiments of the device 1 shown in the illustrations, the container 2 is composed of a half-shell 6 having an access opening to the compartment 3 and a door 7 hinged on one side of the half-shell 6 and mobile for opening/closing the compartment 3.

Usefully, both the half-shell 6 and the door 7 have an outer surface made of insulating material, of the polymer material type or the like.

The half-shell 6, furthermore, comprises a bottom through hole 8 wherein is inserted and comes out the extremity of the bag A having the mouth B for fluid dispensing.

Advantageously, the retention means of the bag A comprise a plate 9 arranged inside the compartment 3, fastened to the half-shell 6 and having a seat designed to accommodate the bag A.

The seat is provided with the above-mentioned heat conductive surface 4 and, in particular, coincides with such heat conductive surface 4 inasmuch as the entire plate 9 is made using a highly heat conductive material such as, e.g., aluminium or the like.

Furthermore, the seat 4 is shaped so as to reproduce the profile and the dimensions of the bag A and, in particular, it has two sloping shoulders 10 in the proximity of the through hole 8, that reproduce the shape of the lower portion of a bag A of conventional type and which are distanced to allow the transit of the extremity of the bag A having the mouth B towards the through hole 8.

The seat 4 defines a substantially sloped surface on which a face of the bag A can be positioned, resting. This makes it possible to keep the bag A in contact with the surface of the seat 4, heated by the heating means 5, during the emptying of the bag itself and by means of the force of gravity only, or under the suction effect of pumps and injectors of various types. To further guarantee maintaining perfect adherence between the bag A and the surface of the seat 4 during the entire phase of administration of the fluid to a patient, the above retention means also comprise a securing element 11 associated with the door 7, positionable in contact with the face of the bag A substantially opposite the seat 4, capable of moving towards the bag A by means of action of elastic means 12, and capable of moving away from bag A against elastic means 12.

In point of fact, once the door 7 is closed on the compartment 3, the securing element 11 is placed in contact with the face of the bag A opposite the seat 4 and, by means of the thrust action of the elastic means 12, keeps the bag A in contact with the heated surface of the seat 4 during emptying.

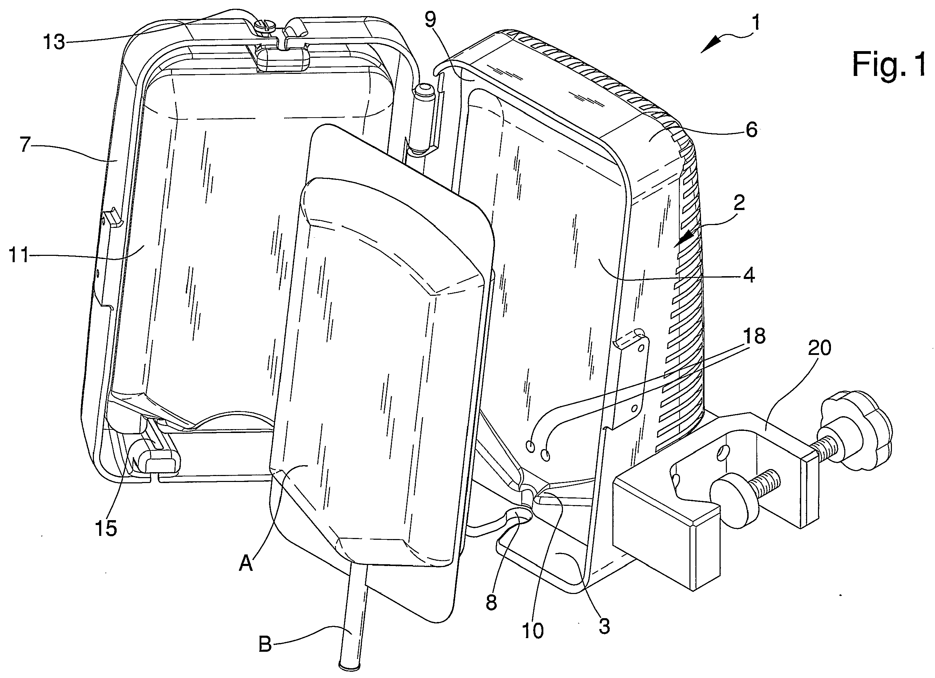

With reference to a first embodiment of the device 1 shown in the figures 1, 2 and 4, the securing element 11 is composed of a substantially plate-shaped body, associated moving with the door 7 by means of guide means and the interposition of the elastic means 12.

In particular, the guide means comprise three screws 13 that can be tightened in corresponding threaded holes 14 on the top and bottom edges of the securing element 11, through respective elongated slots 15 which extend on the bottom and top edges of the door 7.

The guide means also comprise three first tubular elements 16 overhanging from the face of the securing element 11 opposite the face positionable in contact with the bag A. Each of such first tubular elements 16 is fitted sliding and to measure inside corresponding second tubular elements 17 overhanging from the inner surface of the door 7.

The elastic means 12 are composed of three thrust springs housed inside the first and second tubular elements 16 and 17.

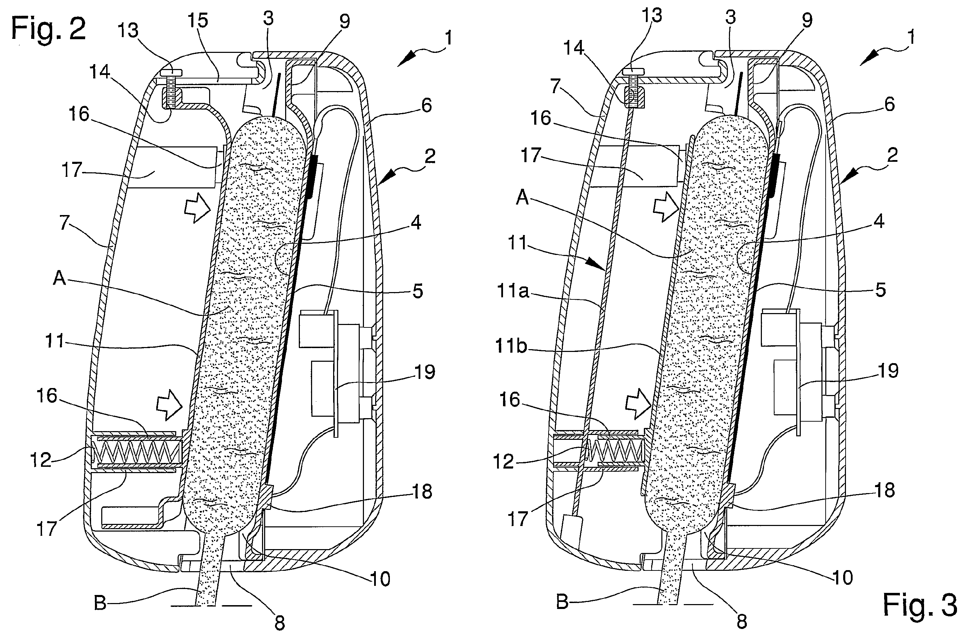

In point of fact, as shown in figure 2, the thrust springs 12 act between the door and the securing element 11, pushing the latter into contact with the bag A arranged inside the seat 4.

With reference to a second embodiment of the device 1 shown in the figure 3, the securing element 11 is composed of a base 11 a that can be fastened to the container by means of one or more screws 13 and a mobile element 11 b, made up of a sheet, which is associated moving with the base 11a by means of guide means and by interposition of the elastic means 12.

In particular, the guide means comprise three first tubular elements 16 overhanging from the face of the sheet 11b opposite the face positionable in contact with the bag A. Each of such first tubular elements 16 is fitted sliding and to measure inside corresponding second tubular elements 17 overhanging from the base 11 a.

The elastic means 12 are composed of three thrust springs 12 housed inside the first and second tubular elements 16 and 17.

In point of fact, as shown in the figure 3, the thrust springs 12 act between the base 11a and the sheet 11b, pushing the latter into contact with the bag A arranged inside the seat 4.

Moreover, further embodiments of the device 1 cannot be ruled out wherein, e.g., it is the plate 9 which is associated moving with the half-shell 6, sliding towards and away from the securing element 11, due to the action of and in contrast to elastic means respectively.

The element 11, being sliding in an axial direction, provides a further advantage. Besides keeping the bag constantly against the heating wall it allows housing at least three different types of bags: 500 ml, 250 ml and 100 ml, which are the most common capacities, in particular for contrast media.

This represents a major aspect because it prevents the operator from having to replace the element 11 when the bag is changed, a change that occurs very often during the course of the day.

Alternatively, it cannot be ruled out that the securing element 11, associated in a removable way with the door 7, have different dimensions and/or conformation according to the type of bag A.

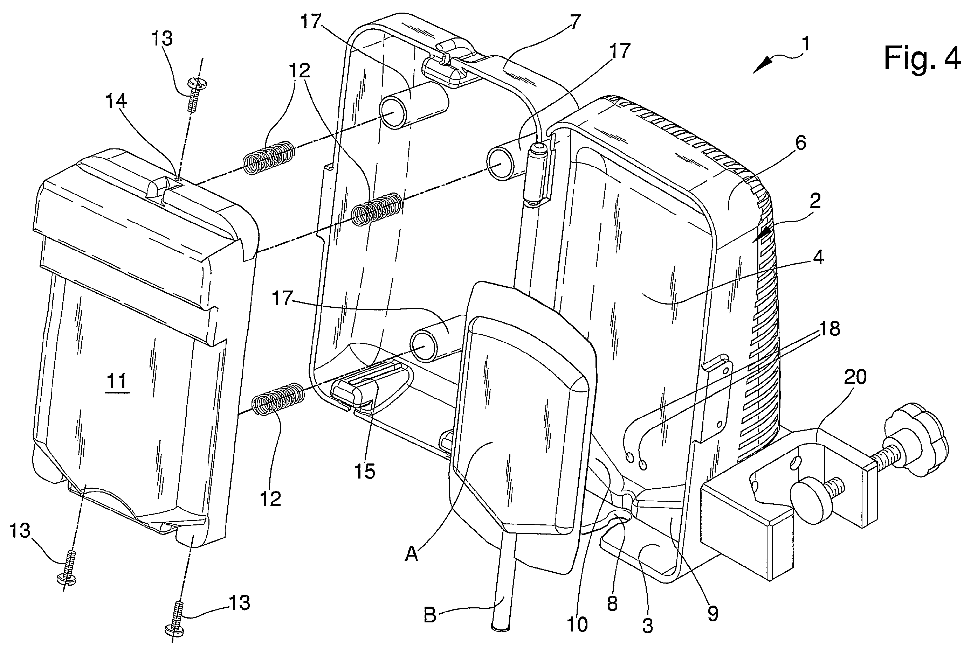

By way of example, but without intending to be exhaustive, figure 4 shows the use of an adequate securing element 11, usable for bags A of small dimensions. The heating means 5 are composed of a substantially plate-shaped heating element, arranged in correspondence to the seat 4 in contact with the face of the plate 9 opposite the face designed to accommodate the bag A.

The heating element 5 can be made up, for example, of one or more heating elements.

Usefully, the device 1 can comprise sight inspection means of the fluid inside the bag A, not shown in the illustration, which can, e.g., be made up of one or more portions in transparent material obtained passing through a side panel of the half-shell 6 or the door 7. Alternatively, the entire door 7 can be made of transparent material.

Such sight inspection means can also comprise one or more LEDs 18, used to check the switch-on and/or the switch-off of the device and/or of the heating means 5 and to display the contents of the bag.

The device 1 also comprises electronic control means 19, made up e.g. of a printed electronic circuit, arranged inside the half-shell 6, behind the plate 9, and connected to the heating element 5 and, if necessary, to the LEDs 18.

The device 1 also comprises gripping means 20 which extend from an outer portion of the half-shell 6 and which are suitable for allowing the fastening to a vertical upright.

The gripping means 20, e.g., can be of the clamp gripping means type.

It has been ascertained how the described invention achieves the proposed objects.

In particular, the fact is underlined that this invention favours the infusion to patients of fluids brought to a preset temperature without the need, during administering, for complicated and cumbersome heating systems or devices. The present invention, furthermore, can be used in conditions of total safety for the patient and utmost operating efficiency.

Another advantage of the present invention is that it is structurally and constructively simple, has limited weight and overall dimensions and is also easy to transport and handle by fairly inexpert health operators.

## Claims

1. Thermostatting device (1) for containers of bio-medical fluids for parenteral administration, particularly bags for medical fluids, blood or the like, comprising:
at least a container (2) having at least one compartment (3) for housing at least one flexible bag (A) containing a bio-medical fluid, said container (2) comprising at least a half-shell (6), and one door (7) associated mobile with said half-shell (6) for closing/opening said compartment (3);
at least one portion of heat conductive surface (4) suitable for being placed in contact with said bag (A) when this is arranged inside said compartment (3); and
heating means (5) for heating said heat conductive surface (4) to bring and/or keep to a preset temperature the fluid inside said bag (A); and retention means (9, 11, 12) for retaining said bag (A) inside said compartment (3) which are suitable for keeping said bag (A) in contact with said heat conductive surface (4) during the emptying of the bag itself;
wherein the retention means (9, 11, 12) comprise at least a plate (9), associated with said container (2), arranged inside said compartment (3), and having at least a seat (4) designed to accommodate said bag (A); the thermostatting device (1) being **characterized by** that it comprises gripping means (20) associated with said container (2) and suitable for allowing it to a vertical upright, and by the fact that said half-shell (6) comprises at least a bottom through hole (8) for inserting an extremity of said bag (A) having the mouth (B) for fluid dispensing; and also by the fact that the seat (4) comprises said heat conductive surface (4) and defines a substantially sloped surface on which a face of the bag (A) can be positioned, resting, so that the bag (A) can be kept in contact with the heated surface of the seat (4) during emptying by means of force of gravity only, while the retention means comprise a securing element (11) associated with the door (7) such that once the door (7) is closed, the securing element (11) is placed in contact with a face of the bag (A) substantially opposite the seat (4), the securing element (11) being capable of moving towards the bag (A) by means of action of elastic means (12), and being capable of moving away from bag (A) against elastic means (12).

2. Device (1) according to claim 1, **characterized by** the fact that said seat (4) is shaped to reproduce at least in part the profile of said bag (A).

3. Device (1) according to one of the preceding claims, **characterized by** the fact that said securing element (11) comprises at least a substantially plate-shaped body (11) associated with said container (2) by placing in between said elastic means (12).

4. Device (1) according to one of the preceding claims, **characterized by** the fact that said securing element (11) comprises at least a base (11a) which can be fastened to said container (2) and at least a mobile element (11b) associated with said base (11a) by placing in between said elastic means (12).

5. Device (1) according to one of the preceding claims, **characterized by** the fact that said securing element (11) is associated with said container (2) in a removable way.

6. Device (1) according to one of the preceding claims, **characterized by** the fact that said plate (9) is made entirely of heat conductive material.

7. Device (1) according to one of the preceding claims, **characterized by** the fact that said heating means (5) comprise at least a heating element (5) arranged in contact with said plate (9), in correspondence to said seat (4).

8. Device (1) according to claim 7, **characterized by** the fact that said heating element (5) has a substantially plate shape and is associated with a face of said plate (9) substantially opposite the face of said plate (9) suitable for being placed in contact with said bag (A).

9. Device (1) according to one of the preceding claims, **characterized by** the fact that said container (2) comprises an outer surface made at least in part of heat insulating material.

10. Device (1) according to claim 9, **characterized by** the fact that said heat insulating material is a polymeric material or the like.

11. Device (1) according to any of claims 6 - 10, **characterized by** the fact that said heat conductive material is a metal or the like.

12. Device (1) according to one of the preceding claims, **characterized by** the fact that it comprises sight inspection means for determining the level of said bio-medical fluid inside said bag (A).

13. Device (1) according to claim 12, **characterized by** the fact that said sight inspection means comprise at least a portion in substantially transparent material obtained through a side panel of said container (2).

14. Device (1) according to claim 12 or 13, **characterized by** the fact that said sight inspection means comprise at least a LED or the like (18) suitable for verifying the switching on and/or the switching off of at least one between said device (1) and said heating means (5) and/or for displaying the contents of said bag (A).

15. Device (1) according to one of the preceding claims, **characterized by** the fact that it comprises control electronic means (19), associated with said container (2) and with said heating means (5) and suitable for controlling the heating of the fluid inside said bag (A).

## Patentansprüche

1. Temperaturregelnde Vorrichtung (1) für Behälter biomedizinischer Fluide zur parenteralen Verabreichung, insbesondere Beutel für medizinische Fluide, Blut oder dergleichen, mit: wenigstens einem Behälter (2) mit wenigstens einem Abteil (3) zur Aufnahme wenigstens eines flexiblen Beutels (A), der ein biomedizinisches Fluid enthält, wobei der Behälter (2) wenigstens eine Halbschale (6) und eine mit der Halbschale (6) beweglich verbundene Tür (7) zum Schließen/Öffnen des Abteils (3) umfasst;
wenigsten einem Bereich mit wärmeleitfähiger Oberfläche (4), der dazu geeignet ist, in Kontakt mit dem Beutel (A) gebracht zu werden, wenn dieser innerhalb des Abteils (3) angeordnet ist; und
eine Heizeinrichtung (5) zum Erwärmen der wärmeleitfähigen Oberfläche (4), um das Fluid innerhalb des Beutels (A) auf eine voreingestellte Temperatur zu bringen und/oder auf dieser zu halten; und eine Halteeinrichtung (9, 11, 12) zum Halten des Beutels (A) innerhalb des Abteils (3), die den Beutel (A) während des Leertingsvorgangs des Beutels mit der wärmeleitfähigen Oberfläche (4) in Kontakt halten kann; wobei die Halteeinrichtung (9, 11, 12) wenigstens eine Platte (9) umfasst, die mit dem Behälter (2) verbunden ist, innerhalb des Abteils (3) angeordnet ist und wenigstens einen Sitz (4) aufweist, der so ausgebildet ist, dass dieser den Beutel (A) aufnimmt; wobei die temperaturregelnde Vorrichtung (1) **dadurch gekennzeichnet ist, dass** diese eine Greifeinrichtung (20) umfasst, die mit dem Behälter (2) verbunden ist und geeignet ist, um diesen an einem vertikalen Ständer anzubringen und durch die Tatsache, dass die Halbschale (6) wenigstens ein Bodendurchgangsloch (8) zum Einführen eines Endes des Beutels (A) umfasst, das den Mund (B) zur Fluidausgabe aufweist; und auch durch die Tatsache, dass der Sitz (4) die wärmeleitende Oberfläche (4) umfasst und eine im wesentlichen schräge Oberfläche bildet, auf welcher eine Fläche des Beutels (A) ruhend positioniert werden kann, so dass der Beutel (A) während des Entleertingsvorgangs ausschließlich mittels Schwerkraft in Kontakt mit der erwärmten Oberfläche des Sitzes (4) gehalten werden kann, wohingegen die Halteeinrichtung eine Sichemngselement (11) umfasst, das mit der Tür (7) derart verbunden ist, dass sobald die Tür (7) geschlossen ist, das Sichemngselement (11) in Kontakt mit der Fläche des Beutels (A) im Wesentlichen gegenüber dem Sitz (4) in Kontakt gebracht wird, wobei das Sichemngselement (11) mit Hilfe der Wirkung einer elastischen Einrichtung (12) in Richtung des Beutels (A) bewegt werden kann und gegen die elastische Einrichtung (12) vom Beutel (A) weg bewegt werden kann.

2. Vorrichtung (1) nach Anspruch 1, **dadurch gekennzeichnet, dass** der Sitz (4) so geformt ist, dass dieser wenigstens teilweise das Profil des Beutels (A) reproduziert.

3. Vorrichtung (1) nach einem der vorstehenden Ansprüche, **dadurch gekennzeichnet, dass** das Sichemngselement (11) wenigstens einen im Wesentlichen plattenförmigen Körper (11) umfasst, der mit dem Behälter (2) verbunden ist, indem dieser zwischen die elastischen Einrichtung (12) gebracht wird.

4. Vorrichtung (1) nach einem der vorstehenden Ansprüche, **dadurch gekennzeichnet, dass** das Sichemngselement (11) wenigstens eine Basis (11a) umfasst, die mit dem Behälter (2) befestigt werden kann, und wenigstens ein bewegliches Element (11b) das mit der Basis (11a) verbunden ist, indem dieses zwischen die elastische Einrichtung (12) gebracht wird.

5. Vorrichtung (1) nach einem der vorstehenden Ansprüche, **dadurch gekennzeichnet, dass** das Sichemngselement (11) mit dem Behälter (2) in einer lösbaren Weise verbunden ist.

6. Vorrichtung (1) nach einem der vorstehenden Ansprüche, **dadurch gekennzeichnet, dass** die Platte (9) vollständig aus wärmeleitfähigem Material hergestellt ist.

7. Vorrichtung (1) nach einem der vorstehenden Ansprüche, **dadurch gekennzeichnet, dass** die Heizeinrichtung (5) wenigstens ein Heizelement (5) umfasst, das in Kontakt mit der Platte (9) angeordnet ist, in Zusammenspiel mit dem Sitz (4).

8. Vorrichtung (1) nach Anspruch 7, **dadurch gekennzeichnet, dass** das Heizelement (5) eine im Wesentlichen Plattenform hat und mit einer Fläche der Platte (9) im Wesentlichen gegenüber der Fläche der Platte (9), die in Kontakt mit dem Beutel (A) gebracht werden kann, verbunden ist.

9. Vorrichtung (1) nach einem der vorstehenden Ansprüche, **dadurch gekennzeichnet, dass** der Behälter (2) eine Außenfläche umfasst, die wenigstens teilweise aus wärmeisolierendem Material hergestellt ist.

10. Vorrichtung (1) nach Anspruch 9, **dadurch gekennzeichnet, dass** das wärmeisolierende Material ein Polymermaterial oder dgl. ist.

11. Vorrichtung (1) nach einem der Ansprüche 6 bis 10, **dadurch gekennzeichnet, dass** das wärmeleitfähige Material ein Metall oder dgl. ist.

12. Vorrichtung (1) nach einem der vorstehenden Ansprüche, **dadurch gekennzeichnet, dass** diese eine Sicht-Inspektionseinrichtung zum Bestimmen des Pegels des biomedizinischen Fluids innerhalb des Beutels (A) umfasst.

13. Vorrichtung (1) nach Anspruch 12, **dadurch gekennzeichnet, dass** die Sicht-Inspektionseinrichtung wenigstens einen Bereich aus im Wesentlichen transparentem Material umfasst, der über ein Seitenfeld des Behälters (2) erhalten wird.

14. Vorrichtung (1) nach Anspruch 12 oder 13, **dadurch gekennzeichnet, dass** die Sicht-Inspektionseinrichtung wenigstens eine LED oder dgl. (18) umfasst, mit der die Einschaltung und/oder die Ausschaltung der Vorrichtung (1) und/oder der Heizeinrichtung (5) verifiziert werden kann und/oder die Inhalte des Beutels (A) angezeigt werden kann.

15. Vorrichtung (1) nach einem der vorstehenden Ansprüche, **dadurch gekennzeichnet, dass** diese eine elektronische Steuereinrichtung (19) umfasst, die mit dem Behälter (2) und mit der Heizeinrichtung (5) verbunden ist und das Erwärmen des Fluids innerhalb des Beutels (A) steuern kann.

## Revendications

1. Dispositif de thermostatisation (1) pour contenants de fluides biomédicaux pour administration par voie parentérale, en particulier des poches pour fluides médicaux, sang ou similaire, comprenant :
au moins un contenant (2) possédant au moins un compartiment (3) servant à loger au moins une poche souple (A) contenant un fluide biomédical, ledit contenant (2) comprenant au moins une demi-coque (6), et une porte (7) associée de façon mobile avec ladite demi-coque (6) pour fermer/ouvrir ledit compartiment (3) ;
au moins une portion de surface conductrice de chaleur (4) apte à être mise en contact avec ladite poche (A) lorsque celle-ci est disposée à l'intérieur dudit compartiment (3) ; et
des moyens de chauffe (5) pour échauffer ladite surface conductrice de chaleur (4) pour amener et/ou maintenir à une température prédéfinie le fluide à l'intérieur de ladite poche (A) ; et des moyens de retenue (9, 11, 12) pour retenir ladite poche (A) à l'intérieur dudit compartiment (3) qui sont aptes à maintenir ladite poche (A) en contact avec ladite surface conductrice de chaleur (4) lors du vidage de la poche proprement dite ;
dans lequel les moyens de retenue (9, 11, 12) comprennent au moins une plaque (9), associée audit contenant (2), agencée à l'intérieur dudit compartiment (3), et possédant au moins un siège (4) conçu pour accueillir ladite poche (A) ; le dispositif de thermostatisation (1) étant **caractérisé en ce qu'**il comprend des moyens de préhension (20) associés audit contenant (2) et aptes à lui permettre d'être en position verticale, et **en ce que** ladite demi-coque (6) comprend au moins un trou traversant inférieur (8) permettant l'insertion d'une extrémité de ladite poche (A) possédant l'embouchure (B) pour l'administration du fluide ; et aussi **en ce que** le siège (4) comprend ladite surface conductrice de chaleur (4) et définit une surface sensiblement inclinée sur laquelle une face de la poche (A) peut être positionnée, reposant en appui, de manière à pouvoir maintenir la poche (A) en contact avec la surface chauffée du siège (4) lors du vidage par la force de gravité seulement, alors que les moyens de retenue comprennent un élément de sécurisation (11) associé à la porte (7) de manière qu'une fois la porte (7) fermée, l'élément de sécurisation (11) est placé en contact avec une face de la poche (A) sensiblement à l'opposé du siège (4), l'élément de sécurisation (11) pouvant se déplacer vers la poche (A) par l'intermédiaire de l'action de moyens élastiques (12), et pouvant s'éloigner de la poche (A) à l'encontre des moyens élastiques (12).

2. Dispositif (1) selon la revendication 1, **caractérisé en ce que** ledit siège (4) est conformé pour reproduire au moins partiellement le profil de ladite poche (A).

3. Dispositif (1) selon l'une des revendications précédentes, **caractérisé en ce que** ledit élément de sécurisation (11) comprend au moins un corps sensiblement en forme de plaque (11) associé audit contenant (2) par interposition desdits moyens élastiques (12).

4. Dispositif (1) selon l'une des revendications précédentes, **caractérisé en ce que** ledit élément de sécurisation (11) comprend au moins une base (11a) qui peut être fixée audit contenant (2) et au moins un élément mobile (11b) associé à ladite base (11a) par interposition desdits moyens élastiques (12).

5. Dispositif (1) selon l'une des revendications précédentes, **caractérisé en ce que** ledit élément de sécurisation (11) est associé audit contenant (2) de manière amovible.

6. Dispositif (1) selon l'une des revendications précédentes, **caractérisé en ce que** ladite plaque (9) est réalisée entièrement en matériau thermoconducteur.

7. Dispositif (1) selon l'une des revendications précédentes, **caractérisé en ce que** lesdits moyens de chauffe (5) comprennent au moins un élément chauffant (5) agencé en contact avec ladite plaque (9), en correspondance avec ledit siège (4).

8. Dispositif (1) selon la revendication 7, **caractérisé en ce que** ledit élément chauffant (5) est sensiblement en forme de plaque et est associé à une face de ladite plaque (9) sensiblement à l'opposé de la face de ladite plaque (9) apte à être mise en contact avec ladite poche (A).

9. Dispositif (1) selon l'une des revendications précédentes, **caractérisé en ce que** ledit contenant (2) comprend une surface externe réalisée au moins partiellement en matériau thermo-isolant.

10. Dispositif (1) selon la revendication 9, **caractérisé en ce que** ledit matériau thermo-isolant est un matériau polymérique ou similaire.

11. Dispositif (1) selon l'une quelconque des revendications 6 - 10, **caractérisé en ce que** ledit matériau thermoconducteur est un métal ou similaire.

12. Dispositif (1) selon l'une des revendications précédentes, **caractérisé en ce qu'**il comprend des moyens de contrôle visuel pour déterminer le niveau dudit fluide biomédical à l'intérieur de ladite poche (A).

13. Dispositif (1) selon la revendication 12, **caractérisé en ce que** lesdits moyens de contrôle visuel comprennent au moins une portion en matériau sensiblement transparent obtenue à travers un panneau latéral dudit contenant (2).

14. Dispositif (1) selon la revendication 12 ou 13, **caractérisé en ce que** lesdits moyens de contrôle visuel comprennent au moins une LED (diode électroluminescente) ou similaire (18) apte à vérifier la mise sous tension et/ou la mise hors tension d'au moins un parmi ledit dispositif (1) et lesdits moyens de chauffe (5) et/ou pour afficher le contenu de ladite poche (A).

15. Dispositif (1) selon l'une des revendications précédentes, **caractérisé en ce qu'**il comprend des moyens électroniques de commande (19), associés audit contenant (2) et auxdits moyens de chauffe (5) et aptes à commander l'échauffement du fluide à l'intérieur de ladite poche (A).
